# EUROPEAN PATENT SPECIFICATION

(11) **EP 1 797 874 B1**
(45) Date of publication and mention of the grant of the patent: **04.11.2015**
(21) Application number: 05714849.6
(22) Date of filing: 16.03.2005
(51) Int. Cl.: A61K 9/50, A61K 31/337, A61P 35/00, A61K 9/16

(54) **VASCULAR EMBOLUS OF PACLITAXEL-SODIUM ALGINATE MICROSPHERE AND ITS PREPARATION**
GEFÄSSEMBOLUS AUS EINEM PACLITAXEL-NATRIUMALGINAT-MIKROKÜGELCHEN UND SEINE HERSTELLUNG
MICROCAPSULES D'ALGINATE DE SODIUM CONTENANT DU PACLITAXEL QUI PERMET DE TRAITER UN EMBOLE VASCULAIRE

(30) Priority: 16.09.2004 CN 200410074464
(43) Date of publication of application: 20.06.2007
(73) Proprietor: Beijing Hongyiyao Science & Technology Development Co. Ltd., Beijing 100088 (CN)
(72) Inventor: LI, Xinjian, Room 211, Building B, Youyan Mansion, Beijing 100088 (CN); HONG, Hong, Room 211, Building B, Youyan Mansion, Beijing 100088 (CN)
(74) Representative: Nevant, Marc
(86) International application number: PCT/CN2005/000320
(87) International publication number: WO 2006/029554

(56) References cited:
- WO-A1-99/59549
- WO-A1-2004/014446
- WO-A2-02/49501
- WO-A2-03/007914
- WO-A2-03/020210
- WO-A2-2004/019999
- CN-A- 1 399 958
- DAI Z. ET AL: 'Studies on cetyl-chitosan nanosphere as carriers for paclitaxel' CHINESE TRADITIONAL AND HERBAL DRUGS vol. 34, no. 2, February 2003, pages 20 - 122, XP008112837
- XIONG X. ET AL: 'Preliminary study on preparation of paclitaxel microsphere' CHINES JOURNAL OF NEW DRUGS vol. 10, no. 7, 2001, pages 517 - 519, XP008138004

## Description

### Field of the Invention

The present invention relates to a kind of sodium alginate microsphere vascular embolus containing paclitaxel and its preparation.

### Background of the Invention

According to data on World Cancer Registration, the incidence of ovarian cancer is the highest in women in Chile, being 21/100 000, while the lowest in Japan, being 3.1/10 000. The great damage to women is obvious. In China, ovarian cancer is also a common disease in gynecology, among which malignant tumor accounts for about 10%. Besides, 70% patients of ovarian cancer are diagnosed at its late stage. The mortality is the first place in malignant tumor of female genitals with 5-year survival rate of 30%-40%, which results in great harm to women's health and life. Unfortunately, the incidence shows a yearly increasing tendency and has increased thrice in recent 40 years. Study shows the cured rate of it by paclitaxel may reach 30%-60%. No surprise it has roused so great attention from many countries and scholars! Experts' research report and clinical application suggest that paclitaxel shows better therapeutic effect on ovarian, uterine and mammary cancer, and also has obvious therapeutic effect on pancreatic cancer, colon carcinoma, prostatic carcinoma, metastatic renal carcinoma, retinal carcinoma, melanoma, tumor of head and neck, etc.

Obvious efficacy has been obtained by microsphere embolotherapy in treatment of hepatic carcinoma, renal carcinoma, prostatic carcinoma, bladder carcinoma, sigmoid colon carcinoma, uterine cervix cancer, ovarian cancer, etc. in foreign countries. It is beneficial to take out medicinal microsphere embolotherapy of tumor in different phases. Pre- and post-operative embolotherapy of resectable tumor that can be excised not only can reduce perioperational hemorrhage, but also can prevent postoperative metastasis and extension. Embolotherapy of unresectable tumor, on one hand, can reduce side effect of general chemotherapy and enhance local therapeutic efficacy, on the other hand, can block tumor vessel and inhibit tumor growth. Sometimes opportunity of tumor excision may be re-obtained.

Treatment of tumor depends on the developing of antitumor drugs to a greater extent. Studies on antitumor drugs are prospering. Since paclitaxel(Taxol) was extracted and purified from bark or leaves of yewtree about 30 years ago, researchers at home and abroad have carried out further studies on anticancer mechanism, range of cancer treatment, dosage and medication of Taxol, how to use with other drugs in combination, etc. Due to its excellent anticancer activity and peculiar anticancer mechanism, paclitaxel is recognized as "a star" in anticancer drugs.

As early as in 1856, Lucas H. extracted powder like alkaline composition i.e. taxine from leaves of European Taxus baccata. But there is no great progress in the following 100 years. In 1958 experts in fields of histochemistry, ecosystem, pharmacology and clinics of USA National Cancer Institute (NCI) screened anticancer activity from 35,000 kinds of plant extracts. In 1964 Chemists Wall and Wani successfully isolated Paclitaxelfrom bark of Taxus baccata. He sequently proved this extract shows high activity to *in vitro* cultured mice tumor cells in cytotoxicity experiment. Wani discovered the chemical formula of Paclitaxol, C₄₇H₅₁O₁₄N. The molecular weight of the natural Paclitaxelis 853.92, its solubility in water is less than 0.004µg/ml, plasma and protein binding ratio is 89-98%, and the average of half-life in human body is in the range of 5.3 h to 17.4h. Paclitaxelcan also be synthesized, the molecular formula, molecular weight, plasma and protein binding ratio as well as the half-life of its synthetic form is C₄₃H₅₃O₁₄N, 807.9, 93-94%, and 11.1h, respectively.

Molecular structure of Paclitaxelis shown as follow:

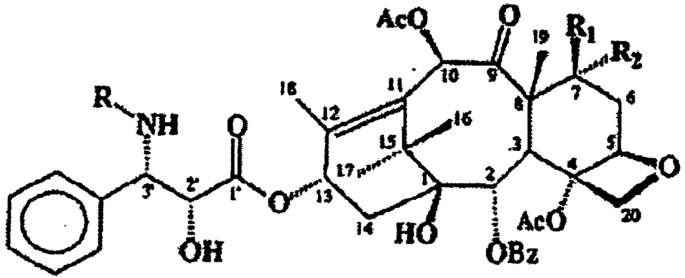

Although paclitaxel shows high activity to mode tumor, its clinical experiment cannot be carried out due to its limited source, and the preparation of it brought about by its insolubility in water. Prof. Susan B. Horwitz and his colleagues in USA Einstein Medical College found peculiar anticancer mechanism of paclitaxel until 1979. Paclitaxel clinical study was conducted and entered in phase I from 1983 to 1987, and phase II from 1987 to 1990, and phase III in 1990. On December 9, 1992, USA FDA formally sanctified paclitaxel as new anticancer drug of late-stage ovarian cancer with trade name of Paclitaxol. It is on market in more than 40 countries in the Europe, America, South Africa, etc. Drug Research Institute of Chinese Academy of Medical Sciences and Haikou Pharmaceutic Factory have obtained new drug certificate.

Paclitaxel has peculiar mechanism, can promote microtubule polymerization and inhibit its depolymerization, inhibit mitosis, block cell proliferation at G₂/M phase, and further induce cell apoptosis, and is a cytotoxicity drug with high performance. Recent studies also show paclitaxel also fights against invasion and metastasis. So it is a kind of new type anticancer drug with great expectations. However, poor water solubility and drug resistance, etc. limit its extensive application. The drug resistance mechanism mainly include □ slightly lower intracellular drug concentration due to reduced intake or increased discharge of cytotoxicity drugs; □ overexpression of P-89 induced by multidrug resistance (MDR) gene, MDR gene related protein (MRP), lung resistance-related protein (LRP); □ obvious increased expression of glutathione metabolism related enzyme in drug resistant strain.

Paclitaxel can make microtubulin and microtubulin dimer constituting microtubulin loss dynamic equilibrium, induce and promote microtubulin polymerization and microtubulin assembly, prevent depolymerization, thus to prevent cancer cells growth. The binding part of paclitaxel is different from that of ATP, catharanthine, colchicine. Paclitaxel binds to 31 amino acids on N terminal of subunit of βmicrotubular protein in microtubule instead of microtubulin dimmer. In contact cells, paclitaxel may induce microtubular fasciculation and make mitotic spindle to form huge amount of astral body, and the former is a useful clinical index for lethal drug. *In vitro* it can inhibit cell division and proliferation to stop at mitosis prophase (G₂ phase) and mitotic period (M phase) most sensitive to radioactivity by cell induction, enhance ion irradiated cytotoxiciy to death, thus to play an anticancer role.

Since 1984 Drug Research Institute of Chinese Academy of Medical Sciences, etc. have investigated and conducted chemical study on Taxus mairei in China, and found rich Taxus baccata resources in Sichuan, Yunnan, Northeast China, etc., from which paclitaxel was extracted. Preclinical chemical and pharmacological studies have been completed. 121 cases of middle- and late-stage cancer patients were treated with paclitaxel according to the co-established phase□clinical trial plan in 10 units of the corporation group. Paclitaxel was generally accepted in medicine field and other associated sectors due to its peculiar anticancer mechanism. But its water insolubility and side effect in clinical application bring about certain difficulty for its application. In recent years people have carried out study and exploration on medicinal dosage form of paclitaxel so as to find a breakthrough to overcome the difficulties mentioned above.

Because paclitaxel itself almost can not been dissolved in water, most clinical paclitaxel preparation is oil preparation made of organic solvent and oil. These medicinal carriers may cause some adverse reactions, so the medication process must be cautiously observed. In recent years people bind some water soluble macromolecular carriers to it to carry it into water. This study opens a new path to solve water solubility of paclitaxel. Generally it is wrapped by lipid body, cyclodextrin, polyglycol and made into emulsion and injectable powder, etc.

At present there is no precedent at home and abroad to take sodium alginate as drug carrier to wrap paclitaxel, and apply it in tumor patients by vascular embolotherapy.

### Summary of the Invention

One objective of the present invention is to provide a kind of safe, nontoxic, non-teratogenic, non-genotoxic, non-carcinogenic sodium alginate microshere vascular embolus containing paclitaxel.

Another objective of the present invention is to provide preparation of sodium alginate microshere vascular embolus containing paclitaxel mentioned above.

The objectives of the present invention are achieved by the following technique protocol.

A kind of sodium alginate microshere vascular embolus containing paclitaxel, which is comprised of sodium alginate as a drug carrier and paclitaxel which is encapsulated by said sodium alginate.

The weight ratio of said sodium alginate and said paclitaxel ranges from 1:1 to 90:1.

The said sodium alginate microshere vascular embolus containing paclitaxel may be microgel particles or microspheres reserving in divalent mental cations curing solution.

The said sodium alginate microshere vascular embolus containing paclitaxel may also be powdery granule.

The diameter of the said microgel particles or microspheres reserving in the curing solution ranges from 200-550µm or 400-750µm or 600-950µm.

The diameter of the said powdery granule ranges from 100-350µm or 200-550µm or 400-750µm.

The preparation of sodium alginate microshere vascular embolus containing paclitaxel consists of the following steps:
(1) Paclitaxel solution is prepared by dissolving a certain rate of paclitaxel with organic solvent;
(2) Sodium alginate solution is prepared by dissolving a certain rate of sodium alginate;
(3) Curing solution is prepared by dissolving calcium chloride or barium chloride with the concentration of 1-10%;
(4) Paclitaxel solution is mixed with sodium alginate solution, then the mixture is added dropwise to bivalent metal cationic solution under a high-voltage electrostatic droplets device to form sodium alginate microspheres or microgel particles containing Paclitaxel.

The high-voltage electrostatic droplets device comprises an electrostatic device, said electrostatic device has positive and negative electrodes. The positive electrode is connected with a needle of a micro-syringe device and the negative electrode is connected with a stainless steel wires emerged in the bivalent metal cationic curing solution. The mixture solution of palitaxel and sodium alginate in the micro-syringe device is dripped into the bivalent cationic curing solution to form microspheres.

The obtained sodium alginate microshere vascular embolus containing paclitaxel is the microsphere reserving in the curing solution, which is called as wet-microsphere, the diameter can range from 200∼50µm, 400~750µm or 600∼50µm.
The obtained sodium alginate microshere vascular embolus containing paclitaxel is decanted, then the lower microgel particles are put into the oven and keeped in a sealed condition. The obtained powdery granules are called dry microspheres the diameters of which may range from 100-350µm or 200-550µm or 400-750µm.
By adopting intervention radiotherapy or intervention ultrasound, the duct is inserted into feeding artery of target organ to conduct arteriography. The selected diameter of embolus microsphere is determined according to the impression of arteriography. Aseptically open the bottle and leave it until the microspheres are settled at the bottom of the bottle. Remove the curing solution by a syringe then add an equal amount of normal saline to wash the microspheres three times, or remove the curing solution by a syringe then add an equal amount of normal saline, pour normal saline and microsphere into an aseptic bowl. Rinse the microsphere using 50-60 mL normal saline once and discard the rinse solution, and then add in appropriate amount or diluted contrast medium to mix up evenly (leaving microsphere thoroughly floating in the contrast medium), and infuse it slowly or slowly in multiple times depending on concrete conditions by duct under fluoroscopy (Prohibit to overdose of embolism.) till flow rate of contrast medium is obviously reduced, i.e. embolism is finished. Arteriography is conducted again to judge the effect of embolism.
If the sodium alginate microshere vascular embolus containing paclitaxel is powdery granule, then the dry microsphere reserving in sealed container are dissolved in normal saline to swell (wet microsphere), then appropriate amount or diluted contrast medium is added to mix evenly (leaving microsphere thoroughly floating in the contrast medium), and then the mixture is infused slowly or slowly in multiple times in the vessel of the affected portion to embolism ultraselectively by duct under the monitor of image documentation equipment (Over-embolization is absolutely prohibited.) till the flow rate of contrast medium is obviously reduced, i.e. embolism is finished. Arteriography is conducted again to judge the effect of embolism.
The following is the further illustration of the present invention in embodiments, but it does not mean any limitation for the protection range of it.

### Detailed Description of the Invention

### Example 1:

Preparation of sodium alginate microsphere vascular embolus containing paclitaxel
1. Preparation before wrapping
   □ Disposal of glass utensil:
      The clean glass utensil is dried then keeped in oven at 300°Cfor 3 hours (the heat source is removed till the bacteria are killed);
   □ Preparation of paclitaxel solution:
      1.5 kg paclitaxel available in market is weighed and placed in the glass utensil mentioned above, propylene glycol is added dropwise till paclitaxel is completely dissolved to make a paclitaxel solution of 10 mol/l;
   □ Preparation of sodium alginate solution:
      2.0 kg sodium alginate available in market is weighed and placed in the glass utensil, normal saline is added while stirring till sodium alginate is completely dissolved;
   □ Preparation of 1% CaCl₂ solution;
   □ The Paclitaxel solution is mixed with sodium alginate solution;
   □ The mixed solution is extracted by aseptic syringe and added into the calcium chloride solution under the high-voltage electrostatic droplet device. The sodium alginate microsphere containing Paclitaxel settles at the bottom of the glassware with diameter of 200~550µm.

The upper solution is decanted and the lower microgel particles are put into the oven and keeped in a sealed condition. The diameters of dry-microspheres are 100~350µm. Prior to use, the dryp-microspheres should be rehydrated with normal saline for a couple of minutes.

Alternatively, the wet-microspheres can be used directly after the decanter of the upper solution and washed twice.
As for patients with myometrial gland, by adopting intervention radiotherapy or intervention ultrasound, insert the duct into feeding artery of target organ to conduct arteriography. Paclitaxel-containing sodium alginate microsphere with diameter ranging from 200-550µm is selected according to the impression of arteriography. Try to use micro-duct to carry out ultraselective embolus with aseptic operation. Extract CaCl₂ solution from the bottle using injector and add in equal volume normal saline to rinse paclitaxel-containing sodium alginate microsphere (wet ball) for 3 times, or extract the described CaCl₂ solution from the bottle and add in equal volume normal saline, pour normal saline and microsphere into an aseptic bowl. Rinse the microsphere using 50-60 mL normal saline once and discard the rinse solution, and then add in appropriate amount or diluted contrast medium to mix up evenly (leaving microsphere thoroughly floating in the contrast medium), and infuse it into the foci slowly or slowly in multiple times depending on concrete conditions by duct under fluoroscopy (Prohibit to overdose of embolism.) till flow rate of contrast medium is obviously reduced, i.e. embolism is finished. Arteriography is conducted again to judge the effect of embolism.

### Example 2:

Preparation of sodium alginate microsphere vascular embolus containing paclitaxel
1. Preparation before wrapping
   □ Disposal of glass utensil:
      The clean glass utensil is dried then keeped in oven at 300°C for 3 hours (the heat source is removed till the bacteria are killed);
   □ Preparation of paclitaxel solution:
      2 kg paclitaxel available in market is weighed and placed in the glass utensil mentioned above, normal saline and ethanol are added dropwise till paclitaxelis completely dissolved;
   □ Preparation of sodium alginate solution:
      20 kg sodium alginate available in market is weighed and placed in the glass utensil, normal saline is added while stirring till sodium alginate is completely dissolved;
   □ Preparation of 10% CaCl₂ solution;
   □ The Paclitaxel solution is mixed with sodium alginate solution;
   □ The mixed solution is extracted by aseptic syringe and added into the calcium chloride solution under the high-voltage electrostatic droplet device. The sodium alginate microspheres containing Paclitaxel settles at the bottom of the glassware with diameters of 400~750µm.

Alternatively, the wet-microspheres can be used directly after the decanter of the upper solution and washed twice.
As for patients with hysteromyoma, by adopting intervention radiotherapy or intervention ultrasound, insert the duct into feeding artery of target organ to conduct arteriography. Paclitaxel-containing sodium alginate microsphere with diameter ranging from 400-750µm is selected according to the impression of arteriography. Try to use micro-duct to carry out ultraselective embolus with aseptic operation. Extract CaCl₂ solution from the bottle using injector and add in equal volume normal saline to rinse paclitaxel-containing sodium alginate microsphere (wet ball) for 3 times, or extract the described CaCl₂ solution from the bottle and add in equal volume normal saline, pour normal saline and microsphere into an aseptic bowl. Rinse the microsphere using 50-60 mL normal saline once and discard the rinse solution, and then add in appropriate amount or diluted contrast medium to mix up evenly (leaving microsphere thoroughly floating in the contrast medium), and infuse it into the foci slowly or slowly in multiple times depending on concrete conditions by duct under fluoroscopy (Prohibit to overdose of embolism.) till flow rate of contrast medium is obviously reduced, i.e. embolism is finished. Arteriography is conducted again to judge the effect of embolism.

### Example 3:

Preparation of sodium alginate microsphere vascular embolus containing paclitaxel
1. Preparation before wrapping
   □ Disposal of glass utensil:
      The clean glass utensil is dried then keeped in oven at 300°C for 3 hours (the heat source is removed till the bacteria are killed);
   □ Preparation of paclitaxel solution:
      2 kg paclitaxel available in market is weighed and placed in the glass utensil mentioned above, acetone is added dropwise till paclitaxelis completely dissolved;
   □ Preparation of sodium alginate solution:
      150kg sodium alginate available in market is weighed and placed in the glass utensil, normal saline is added while stirring till sodium alginate is completely dissolved;
   □ Preparation of 6% CaCl₂ solution;
   □ The Paclitaxel solution is mixed with sodium alginate solution;
   □ The mixed solution is extracted by aseptic syringe and added into the calcium chloride solution under the high-voltage electrostatic droplet device. The sodium alginate microspheres containing Paclitaxel settles at the bottom of the glassware with diameters of 600∼950µm.

The upper solution is decanted and the lower microgel particles are put into the oven and keeped in a sealed condition. The diameters of dry-microspheres are 400∼750µm. Prior to use, the dryp-microspheres should be rehydrated with normal saline for a couple of minutes.

Alternatively, the wet-microspheres can be used directly after the decanter of the upper solution and washed twice.
As for patients with hysteromyoma, by adopting intervention radiotherapy or intervention ultrasound, insert the duct into feeding artery of target organ to conduct arteriography. Paclitaxel-containing sodium alginate microsphere with diameter ranging from 600-750µm is selected according to the impression of arteriography. Try to use ultraselective micro-duct to carry out embolus with aseptic operation while using. Extract CaCl₂ solution from the bottle using injector and add in equal volume normal saline to rinse paclitaxel-containing sodium alginate microsphere (wet ball) for 3 times, or extract the described CaCl₂ solution from the bottle and add in equal volume normal saline, pour normal saline and microsphere into an aseptic bowl. Rinse the microsphere using 50-60 mL normal saline once and discard the rinse solution, and then add in appropriate amount or diluted contrast medium to mix up evenly (leaving microsphere thoroughly floating in the contrast medium), and infuse it into the foci slowly or slowly in multiple times depending on concrete conditions by duct under fluoroscopy (Prohibit to overdose of embolism.) till flow rate of contrast medium is obviously reduced, i.e. embolism is finished. Arteriography is conducted again to judge the effect of embolism.

### Industrial Application

Sodium alginate, used as a drug carrier in this invention is natural extracts, which is a kind of polysaccharide sodium salt composed by β-D-mannitol and α-L-gulose extracted from natural brown algae. As a kind of linear macromolecule, its molecular weight is 50,000-100,000 Dalton. It has high hydrophilicity and can be easily dissolved in water forming viscous colloid. Cross-link and solidification among macromolecular links may occur under the action of calcium ion. It can be processed into solid spherical or spherical like microsphere in different sizes according to clinical necessity. This kind of microsphere has good biocompatibility. In vivo the living organism, calcium ion may be gradually educed, and microsphere is nontoxicly degraded within 3-6 months due to molecular chain scission. No debris is produced during degradation, and it may also result in the eternal vascular embolism in target organs (When embolus remains in the vessel as long as 2 months, the intravascular thrombus may form and achieve the objective of eternal embolism.), thus to achieve the goal of treatment. In practical operation, using this kind of "biological multifunctional microsphere" embolism materials, by physical clogging tumor or arteriole vessel around the treated portion, may cause vascular closure, block blood supply and nutrients of the tissue in that portion, thus to induce atrophy and necrosis due to 1 ischemia and hypoxia. Meanwhile, it may provide advantageous condition for operation by reducing blood supply of target organs. Therefore, it shows dual therapeutic effect of embolism and drugs by taking this microsphere as drug carriers in treatment of gynecological diseases to slowly release to the tissue of local foci in a timing, positioning, and orientation way, thus to improve therapeutic efficacy greatly.

In the present invention, sodium alginate is selected as carriers added with antitumor target medicine, locally releasnig target medicine in a timing, positioning, and orientation way to kill tumor cells and arrive at treatment objective in light of peculiar anticancer mechanism and clinical application of paclitaxel, semi interpenetrating network structure and degradable principle of sodium alginate microsphere by combining previous clinical practice of sodium alginate microsphere embolus, taking safety, nontoxicity, non-immunogenicity, no inherent toxicity, nontoxic in generation, non-carcinogenicity, etc. into consideration. In the study of sodium alginate microshere vascular embolus containing paclitaxel, paclitaxel is not dissolved in water, the packed paclitaxel droplet will not precipitate and form no microsphere with no crystal educed. These procedures are completed in the study. After adding special mixture reagent, organic solvent, etc., adjusting concentration, frequency and volecity, the microsphere, very well packed is even, round and smooth with evenly scattering medicine. The medicine is carried by microsphere to protect medicinal active group to maintain *in vivo* stability in internal environment, avoid the leakage of paclitaxel from human body too early and too rapidly, thus to arrive at the requirements for clinical application.

Sodium alginate microshere vascular embolus containing paclitaxel in the present invention has become the most promising target releasing medicine system attributable to large carrier amount of medicinal microsphere, longer *in vivo* residence time, target exclusive. When the concentration of paclitaxel is 10 mol/L, no paclitaxel crystal or medicinal microsphere accumulation in the produced medicinal microsphere has been observed by phase contrast microscope. Besides, it still keeps good physical and chemical stability at 4□ for 30 days. When the concentration is 30 mol/L, most paclitaxel crystal and medicinal microsphere accumulation can be observed under microscope. Microsphere granule surface made of sodium alginate has certain negative charge, rejecting each other among granules. In application, dosage should be selected by the foci. Intervention radiotherapy or intervention ultrasound may be adopted to insert the duct into feeding artery of target organ. Mix the medicinal microsphere and contrast medium using injector after arteriography to slowly inject. It will not aggregate in the duct and block the duct.

## Claims

1. Paclitaxel-containing sodium alginate microspheres, which comprise sodium alginate as a drug carrier and paclitaxel, said paclitaxel being encapsulated by said sodium alginate.

2. The microspheres as in claim 1, wherein the weight ratio of said sodium alginate and said paclitaxel ranges from 1:1 to 90:1.

3. The microspheres as in claim 1 or 2, which are in the form of microgel particles or of microspheres kept in a divalent metal-cations curing solution.

4. The microspheres as in claim 1 or 2, which are in the form of powdery granules.

5. The microspheres as in claim 3, wherein the diameter of the said microgel particles or microspheres ranges from 300-550 µm or 500-750 µm or 700-950 µm.

6. The microspheres as in claim 4, wherein the diameter of the said powdery granules ranges from 100-350 µm or 200-550 µm or 400-750 µm.

7. A method for the preparation of the microspheres as defined in claim 1, comprising the following steps:
(1) A paclitaxel solution is prepared by dissolving a certain rate of paclitaxel with an organic solvent;
(2) A sodium alginate solution is prepared by dissolving a certain rate of sodium alginate;
(3) A curing solution is prepared by dissolving calcium chloride or barium chloride at a concentration of 1-10%;
(4) The paclitaxel solution is mixed with the sodium alginate solution, then the mixture is added dropwise to the divalent metal cationic curing solution using a high-voltage electrostatic droplets device to form paclitaxel-containing sodium alginate microspheres or microgel particles.

8. The method as in claim 7, wherein the high-voltage electrostatic droplets device comprises an electrostatic device, said electrostatic device has positive and negative electrodes; the positive electrode is connected with a needle of a micro-syringe device and the negative electrode is connected with a stainless steel wire emerged in the divalent metal cationic curing solution and the mixture solution of paclitaxel and sodium alginate in the micro-syringe device is dripped into the divalent cationic curing solution to form microspheres.

9. The method as in claim 7 or 8, wherein said microspheres are dried to obtain powdery granules.

10. The microspheres as defined in any one of claims 1 to 6, for use in the treatment of tumors, wherein said microspheres are administered in a contrast medium.

## Patentansprüche

1. Paclitaxelhaltige Natriumalginat-Mikrokügelchen, die Natriumalginat als einen Arzneimittelträger und Paclitaxel umfassen, wobei Paclitaxel durch das Natriumalginat verkapselt ist.

2. Mikrokügelchen nach Anspruch 1, wobei das Gewichtsverhältnis von Natriumalginat und Paclitaxel von 1:1 bis 90:1 reicht.

3. Mikrokügelchen nach Anspruch 1 oder 2, die in der Form von Mikrogelpartikeln oder Mikrokügelchen vorliegen, die in einer zweiwertigen Metallkationenaushärtungslösung gelagert werden.

4. Mikrokügelchen nach Anspruch 1 oder 2, die in der Form von pulverförmigem Granulat vorliegen.

5. Mikrokügelchen nach Anspruch 3, wobei der Durchmesser der Mikrogelpartikel oder Mikrokügelchen von 300-550 µm oder 500-750 µm oder 700-950 µm reicht.

6. Mikrokügelchen nach Anspruch 4, wobei der Durchmesser des pulverförmigen Granulats von 100-350 µm oder 200-550 µm oder 400-750 µm reicht.

7. Verfahren zur Herstellung der Mikrokügelchen nach Anspruch 1, umfassend die folgenden Schritte:
(1) eine Paclitaxellösung wird durch Lösen einer bestimmten Menge Paclitaxel in einem organischen Lösungsmittel hergestellt,
(2) eine Natriumalginatlösung wird durch Lösen einer bestimmten Menge Natriumalginat hergestellt,
(3) eine Aushärtungslösung wird durch Lösen von Calciumchlorid oder Bariumchlorid mit einer Konzentration von 1-10 % hergestellt,
(4) die Paclitaxellösung wird mit der Natriumalginatlösung gemischt, dann wird die Mischung tröpfchenweise zur zweiwertigen Metallkationenaushärtungslösung unter Verwendung einer hochspannungselektrostatischen Tröpfchenvorrichtung hinzugegeben, um paclitaxelhaltige Natriumalginat-Mikrokügelchen oder -Mikrogelpartikel zu bilden.

8. Verfahren nach Anspruch 7, wobei die hochspannungselektrostatische Tröpfchenvorrichtung eine elektrostatische Vorrichtung umfasst, wobei die elektrostatische Vorrichtung positive und negative Elektroden aufweist, wobei die positive Elektrode mit einer Nadel einer Mikroinjektionsspritzenvorrichtung verbunden ist und die negative Elektrode mit einem Edelstahldraht verbunden ist, der in die zweiwertige Metallkationenaushärtungslösung hineinragt, und die Mischlösung aus Paclitaxel und Natriumalginat in der Mikroinjektionsspritzenvorrichtung in die zweiwertige Kationenaushärtungslösung getropft wird, um Mikrokügelchen zu bilden.

9. Verfahren nach Anspruch 7 oder 8, wobei die Mikrokügelchen getrocknet werden, um pulverförmiges Granulat zu erhalten.

10. Mikrokügelchen nach einem der Ansprüche 1 bis 6 zur Verwendung bei der Behandlung von Tumoren, wobei die Mikrokügelchen in einem Kontrastmedium verabreicht werden.

## Revendications

1. Microsphères d'alginate de sodium contenant du paclitaxel, comprenant de l'alginate de sodium comme support pharmaceutique et du paclitaxel, ledit paclitaxel étant encapsulé par ledit alginate de sodium.

2. Microsphères selon la revendication 1, dans lesquelles le rapport en poids dudit alginate de sodium et dudit paclitaxel va de 1/1 à 90/1.

3. Microsphères selon la revendication 1 ou 2, qui se présentent sous la forme de particules de microgel ou de microsphères maintenues dans une solution de durcissement de cations métalliques divalents.

4. Microsphères selon la revendication 1 ou 2, qui se présentent sous la forme de granulés pulvérulents.

5. Microsphères selon la revendication 3, dans lesquelles le diamètre desdites particules de microgel ou microsphères va de 300 à 550 µm ou de 500 à 750 µm ou de 700 à 950 µm.

6. Microsphères selon la revendication 4, dans lesquelles le diamètre desdits granulés pulvérulents va de 100 à 350 µm ou de 200 à 550 µm ou de 400 à 750 µm.

7. Procédé de préparation de microsphères selon la revendication 1, comprenant les étapes suivantes :
(1) une solution de paclitaxel est préparée en dissolvant une certaine quantité de paclitaxel avec un solvant organique ;
(2) une solution d'alginate de sodium est préparée en dissolvant une certaine quantité d'alginate de sodium ;
(3) une solution de durcissement est préparée en dissolvant du chlorure de sodium ou du chlorure de baryum à une concentration de 1 à 10 % ;
(4) la solution de paclitaxel est mélangée avec la solution d'alginate de sodium, puis le mélange est ajouté goutte à goutte à la solution de durcissement cationique de métal divalent en utilisant un dispositif d'émission de gouttelettes électrostatique à haute tension pour former des microsphères ou des particules de microgel d'alginate de sodium contenant du paclitaxel.

8. Procédé selon la revendication 7, dans lequel le dispositif d'émission de gouttelettes électrostatique à haute tension comprend un dispositif électrostatique, ledit dispositif électrostatique a des électrodes positives et négatives ; l'électrode positive est reliée à l'aiguille d'un dispositif à micro-seringue et l'électrode négative est reliée à un fil d'acier inoxydable immergé dans la solution de durcissement cationique de métal divalent et le mélange de solution de paclitaxel et de solution d'alginate de sodium dans le dispositif de micro-seringue est ajouté goutte à goutte dans la solution de durcissement cationique divalente pour former des microsphères.

9. Procédé selon la revendication 7 ou 8, dans lequel lesdites microsphères sont séchées pour obtenir des granulés pulvérulents.

10. Microsphères selon l'une quelconque des revendications 1 à 6, pour utilisation dans le traitement des tumeurs, lesdites microsphères étant administrées dans un milieu de contraste.
